# EUROPEAN PATENT APPLICATION

(11) **EP 1 961 742 A1**
(43) Date of publication of application: **27.08.2008**
(21) Application number: 07102910.2
(22) Date of filing: 22.02.2007
(51) Int. Cl.: C07D 211/06, C07D 211/22, C07D 207/06, A61K 31/40, A61K 31/4418, A61P 25/28, A61P 37/00

(54) **compounds of formula (I) as serine protease inhibitors**

(71) Applicant: Novartis AG, 4002 Basel (CH)
(72) Inventor: The designation of the inventor has not yet been filed
(74) Representative: Vögeli-Lange, Regina

(57) **Abstract**

Compounds of formula (I) and compositions thereof and their use as pharmaceutical agents, in particular as inhibitors of serine proteases.

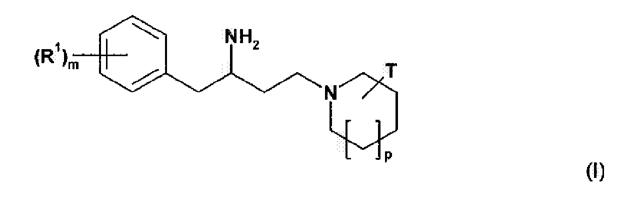

## Description

The present invention relates to novel compounds, in particular novel phenyl propylamine derivatives, and to compositions thereof and to their use as pharmaceutical agents.

The present invention is directed to compounds which are inhibitors of serine proteases, including dipeptidyl peptidases, such as dipeptidyl peptidase-IV and dipeptidyl peptidase-II, for the prevention, delay of progression or the treatment of the human diseases in which serine peptidases are involved.

The present invention is, in particular, directed to compounds which are inhibitors of the dipeptidyl peptidase-IV enzyme ("DPP-IV inhibitors") and which are useful in the treatment or prevention of diseases in which the dipeptidyl peptidase-IV enzyme is involved.

The invention is also directed to pharmaceutical compositions comprising these compounds and the use of these compounds and compositions in the prevention or treatment of such diseases in which the dipeptidyl peptidase-IV enzyme is involved.

More particularly, the compounds of the present invention have the general formula (I) below: 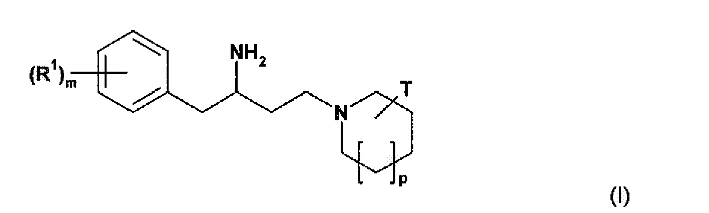
m is 0,1,2,3,4 or 5;
p is 0 or 1;
each R¹ is independently selected from halogen, alkyl, haloalkyl, alkoxy, haloalkyloxy; cycloalkyl; and
T is selected from optionally substituted aryl, optionally substituted alkyl, hydrogen, halogen, alkoxy, haloalkyloxy, COO-alkyl, aryloxy;
and pharmaceutically acceptable salts and N-oxides thereof.

### Definitions

"Alkyl" represents a straight-chain or branched-chain alkyl group, preferably represents a straight-chain or branched-chain, C₁₋₈alkyl particularly preferably represents a straight-chain or branched-chain C₁₋₆alkyl; for example, methyl, ethyl, n- or iso-propyl, n-, iso-, sec- or tert-butyl, n-pentyl, n-hexyl, n-heptyl, n-octyl, n-nonyl, n-decyl, n-undecyl, n-dodecyl, with particular preference given to methyl, ethyl, n-propyl, iso-propyl and tert-butyl. The term alkyl also encompasses "Alkanediyl", which represents a straight-chain or branched-chain alkandiyl group bound by two different carbon atoms to the molecule. Alkyl is optionally substituted by one or more substituents, preferably one to three substituents. The substituents are preferably fluorine, hydroxyl, cyano, (C₁-C₄)-alkoxy, (C₁-C₄)-alkoxycarbonyl, amino, (C₁-C₄)-alkylamino, di-(C₁-C₄)-alkylamino, (C₁-C₄)-alkoxycarbonylamino, or (C₁-C₄)-alkylcarbonylamino. Fluoroalkyl is particularly preferred and is, for example, CF₃, CHF₂, CH₂F, CH₃CHF-, CH₃CF₂-.

"Alkanediyl" preferably represents a straight-chain or branched-chain C₁₋₁₂ alkandiyl, particularly preferably represents a straight-chain or branched-chain C₁₋₆alkanediyl; for example, methanediyl (-CH₂-), 1,2-ethanediyl (-CH₂-CH₂-), 1,1-ethanediyl ((-CH(CH₃)-), 1,1-, 1,2-, 1,3-propanediyl and 1,1-, 1,2-, 1,3-, 1,4-butanediyl, with particular preference given to methanediyl, 1,1-ethanediyl, 1,2-ethanediyl, 1,3-propanediyl, 1,4-butanediyl.

"Cycloalkyl" represents an optionally substituted saturated alicyclic moiety having from three to six carbon atoms. The group may be a polycyclic ring system. This term refers to groups such as cyclopropyl, cyclobutyl, cyclopentyl, cyclohexyl and the like. The group may be optionally substituted with one or more substituents, the substituents being the same or different and selected from fluorine, hydroxyl, cyano, (C₁-C₄)-alkoxy, (C₁-C₄)-alkoxycarbonyl, amino, (C₁-C₄)-alkylamino, di-(C₁-C₄)-alkylamino, (C₁-C₄)-alkoxycarbonylamino, or (C₁-C₄)-alkylcarbonylamino and the like.

Each alkyl part of "alkoxy", "alkoxyalkyl", "alkoxycarbonyl", "alkoxycarbonylalkyl" and "halogenalkyl" and so on shall have the same meaning as described in the above-mentioned definition of "alkyl", especially regarding linearity, saturation, preferential size, and optional substitution.

"Aryl" represents an aromatic hydrocarbon group, preferably a C₆₋₁₀ aromatic hydrocarbon group; for example phenyl, naphthyl, especially phenyl. Aryl is preferably phenyl, naphthyl or 5- to 10-membered heteroaryl, more preferably phenyl or 5- to 6-membered heteroaryl, Aryl is optionally substituted, preferably un-, mono-, di- or trisubstituted. Substituents are preferably halogen, nitro, cyano, formyl, carboxamido, hydroxyl, amino, (C₁-C₄)-alkylamino, di-(C₁-C₄)-alkylamino, (C₁-C₄)-alkyl, (C₁-C₄)-haloalkyl, (C₁-C₄)-alkoxy, (C₁-C₄)-haloalkoxy , (C₁-C₄)-alkoxycarbonyl, (C₁-C₄)- alkanesulfonyl, (C₁-C₄)- alkyl-carbonyl, (C₁-C₄)-alkoxycarbonylamino, or (C₁-C₄)-alkylcarbonylamino.

"Halogen" represents fluoro, chloro, bromo or iodo, preferably represents fluoro, chloro or bromo and particularly preferably represents fluoro.

Salts are preferably physiologically acceptable salts, formed, as applicable, by the addition of an acid or base.

In one embodiment of the present invention, there are provided compounds of formula (II): 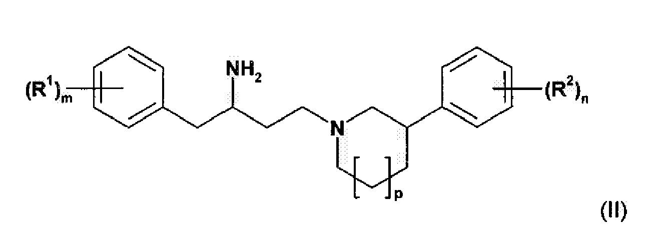 wherein:
n and m are each independently 0,1,2,3,4 or 5;
p is 0 or 1;
each R¹ is independently selected from halogen, alkyl, haloalkyl, alkoxy, haloalkyloxy; cycloalkyl; and
each R² is independently selected from halogen, alkyl, haloalkyl, alkoxy, haloalkyloxy, COO-alkyl, aryloxy; and
   where n is 2 or more, two R² substituents may together form a 5 or 6 membered ring;
   and pharmaceutically acceptable salts and N-oxides thereof.
p is preferably 1.
R² is preferably independently selected from halogen, alkyl, haloalkyl, alkoxy, haloalkyloxy, COO-alkyl, aryloxy.
R² is preferably independently selected from -CF₃ or -O-CF₃.
n is preferably 1 or 2.

In one embodiment of the present invention, there are provided compounds of formula (III): 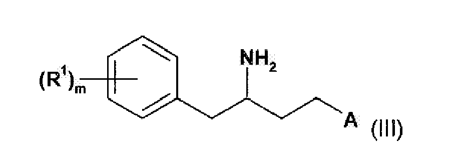 where R¹ and m are as herein before described; and
where A is selected from

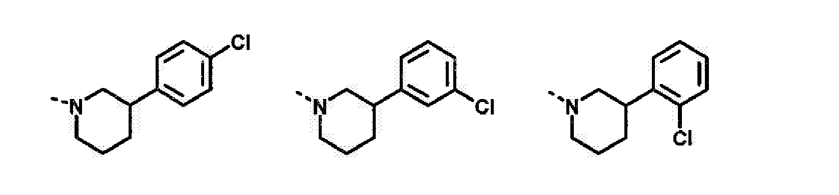

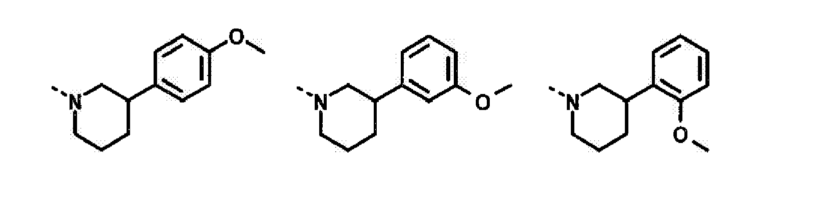

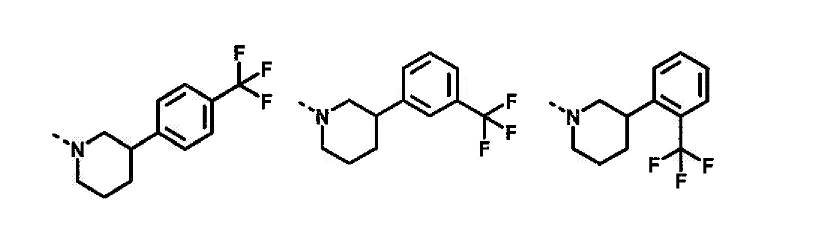

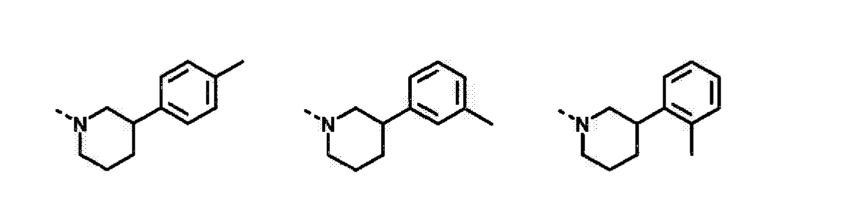

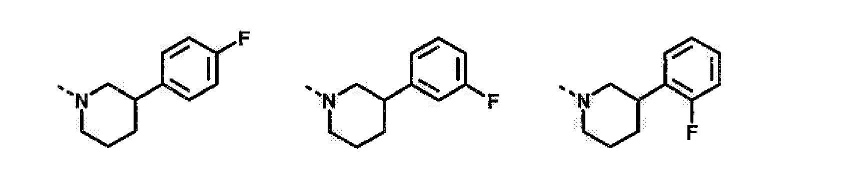

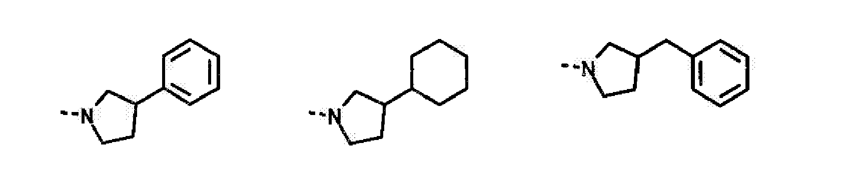

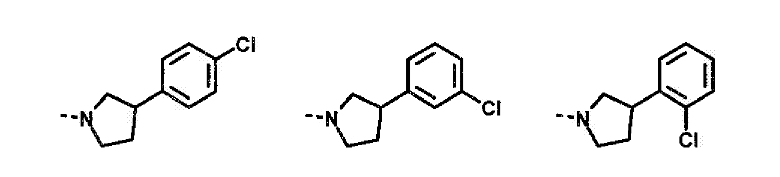

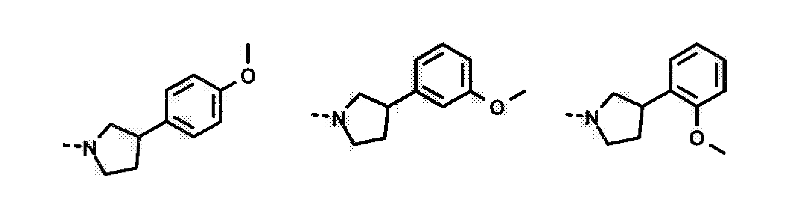

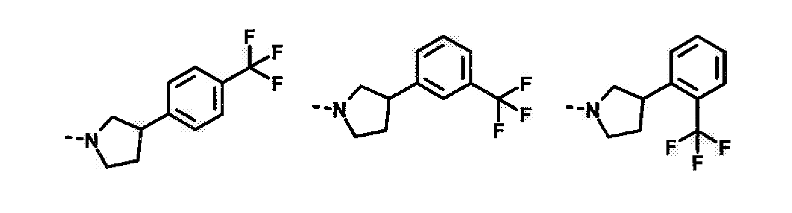

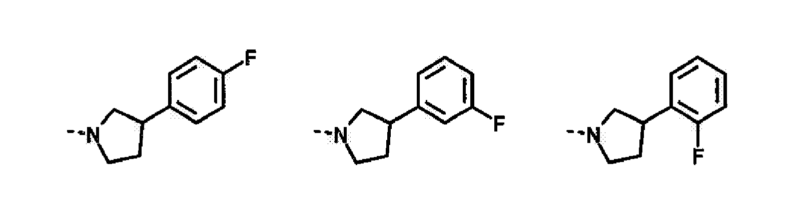

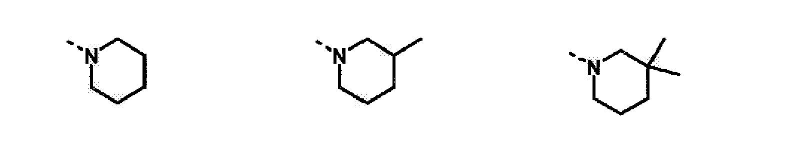

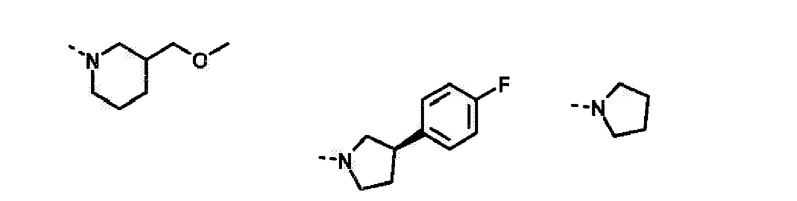

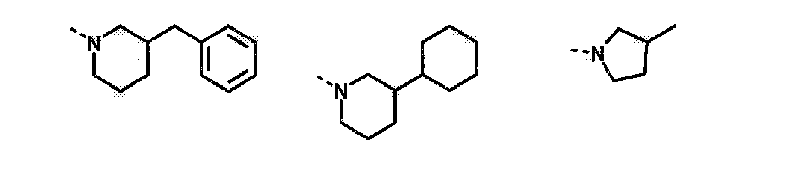

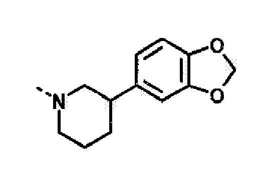

In one class of compounds, m is 3.

In another class of compounds n is 1, 2 or 3.

In a second embodiment of the present invention, there are provided compounds of Formula (IV): 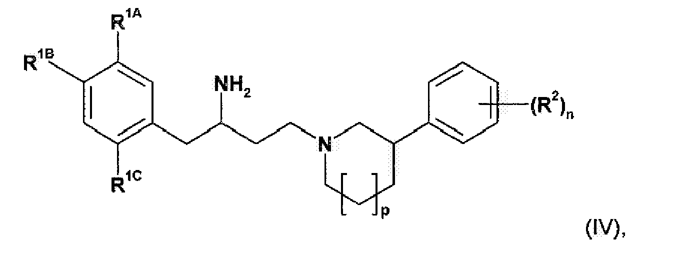 where R^{1A}, R^{1B} and R^{1C} are each independently selected from halogen, alkyl, haloalkyl, alkoxy, haloalkyloxy; cycloalky; and
R², n and p are as hereinbefore defined.
p is preferably 1.

In a third embodiment of the present invention, there are provided compounds of Formula (V): 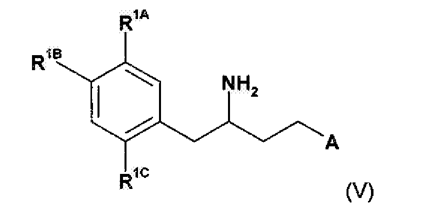 where A, R^{1A}, R^{1B} and R^{1C} are as hereinbefore described.

Preferably, R^{1A}, R^{1B} and R^{1C} are each independently selected from fluorine, alkyl or haloalkyl.

Particularly preferably, R^{1A}, R^{1B} and R^{1C} are all fluoro.

Compounds of Formulae (I) - (V) exist in free form, as a salt or as zwitterion. In this specification, unless otherwise indicated, language such as "compounds of Formulae (I) - (IV)" is to be understood as embracing the compounds in any form, for example free base or acid addition salt form. Salts which are unsuitable for pharmaceutical uses but which can be employed, for example, for the isolation or purification of free compounds of Formulae (I) - (V), such as picrates or perchlorates, are also included. For therapeutic use, only pharmaceutically acceptable salts or free compounds are employed (where applicable in the form of pharmaceutical preparations), and are therefore preferred. Salts are preferably physiologically acceptable salts, formed, as applicable, by the addition of an acid or base.

Compounds of Formulae (I) - (V) may exist in the form of various tautomers. For example, the compounds of Formulae (I) - (V) may show keto-enol-tautomerism. In this specification, the drawing of one possible tautomer includes other possible tautomers as well. The tautomers of the compounds of Formulae (I) - (V) are also embraced by the invention.

Compounds of Formulae (I) - (V) may exist in the form of various zwitterions. For example, the compounds of Formulae (I) - (V) may show protonated amino-groups and deprotonated carboxy-groups.

The compounds of Formulae (I) - (V) may exist in optically active form or in form of mixtures of optical isomers, e.g. in form of racemic mixtures or diastereomeric mixtures. In particular, asymmetrical carbon atom(s) may be present in the compounds of Formulae (I) - (V) and their salts. All optical isomers and their mixtures, including the racemic mixtures, are embraced by the invention.

One or more functional groups, for example carboxy, hydroxy, amino, or mercapto, may need to be protected in the starting materials by protecting groups.

With regard to the use of protecting as mentioned herein, the formation of salts, e.g. for the purposes of purification, and the synthesis of isomers the following considerations may apply:

Protecting groups: In the reaction steps described above, one or more functional groups, for example carboxy, hydroxy, amino, or mercapto, may need to be protected in the starting materials by protecting groups. The protecting groups employed may already be present in precursors and should protect the functional groups concerned against unwanted secondary reactions, such as acylations, etherifications, esterifications, oxidations, solvolysis, and similar reactions. It is a characteristic of protecting groups that they lend themselves readily, i.e. without undesired secondary reactions, to removal, typically by solvolysis, reduction, photolysis or also by enzyme activity, for example under conditions analogous to physiological conditions, and that they are not present in the end-products. The specialist knows, or can easily establish, which protecting groups are suitable with the reactions mentioned hereinabove and hereinafter. The protection of such functional groups by such protecting groups, the protecting groups themselves, and their removal reactions are described for example in standard reference works, such as J. F. W. McOmie, "Protective Groups in Organic Chemistry", Plenum Press, London and New York 1973, in T. W. Greene and P. G. M. Wuts, "Protective Groups in Organic Synthesis", Third edition, Wiley, New York 1999, in "The Peptides"; Volume 3 (editors: E. Gross and J. Meienhofer), Academic Press, London and New York 1981, in "Methoden der organischen Chemie" (Methods of organic chemistry), Houben Weyl, 4th edition, Volume 15/1, Georg Thieme Verlag. Stuttgart 1974, in H.-D. Jakubke and H. Jescheit, "Aminosäuren, Peptide, Proteine" (Amino acids, peptides, proteins), Verlag Chemie, Weinheim, Deerfield Beach, and Basel 1982, and in Jochen Lehmann, "Chemie der Kohlenhydrate: Monosaccharide und Derivate" (Chemistry of carbohydrates: monosaccharides and derivatives), Georg Thieme Verlag, Stuttgart 1974.

Acid addition salts may be produced from the free bases in known manner, and vice-versa. Compounds of formula I in optically pure form can be obtained from the corresponding racemates according to well-known procedures, e.g. HPLC with chiral matrix or via salt formation with a chiral and optically pure couterion, followed by crystallization or chromatography. Alternatively, optically pure starting materials can be used.

Compounds of Formulae (I) - (IV) in optically pure form can be obtained from the corresponding racemates according to well-known procedures, e.g. HPLC with chiral matrix or via salt formation with a chiral and optically pure couterion, followed by crystallization or chromatography. Alternatively, optically pure starting materials can be used. Stereoisomeric mixtures, e.g. mixtures of diastereomers, can be separated into their corresponding isomers in a manner known *per se* by means of suitable separation methods. Diastereomeric mixtures for example may be separated into their individual diastereomers by means of fractionated crystallization, chromatography, solvent distribution, and similar procedures. This separation may take place either at the level of a starting compound or in a compound of formula I itself. Enantiomers may be separated through the formation of diastereomeric salts, for example by salt formation with an enantiomer-pure chiral acid, or by means of chromatography, for example by HPLC, using chromatographic substrates with chiral ligands.

### Synthesis

Compounds of Formula (I) may be synthesised using the following generic scheme:

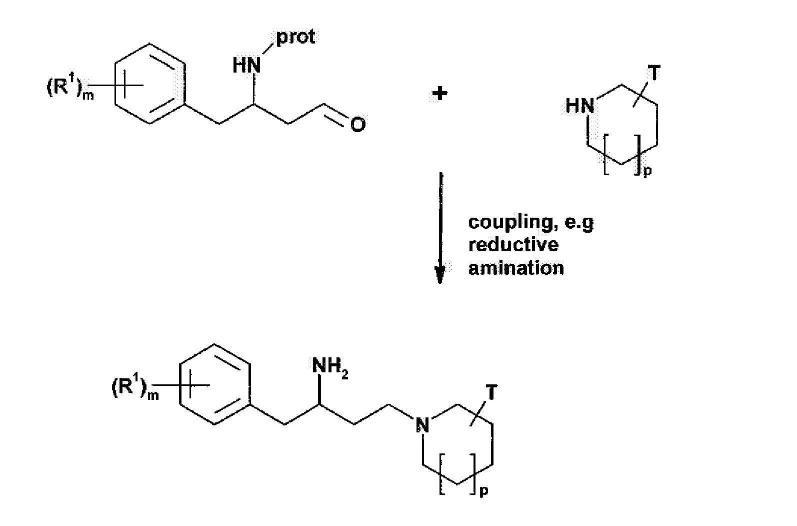

Similarly, for the compounds of formula (II), the above reaction scheme also applies:

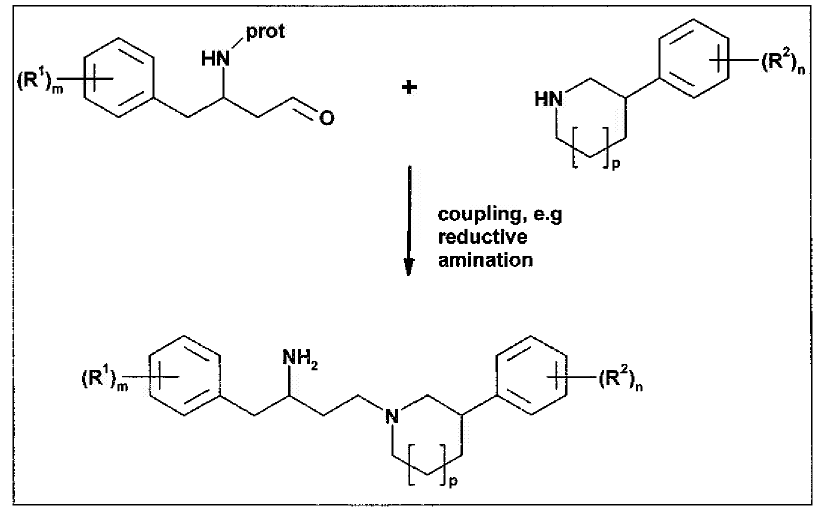

A more specific synthesis is given in the reaction scheme below::

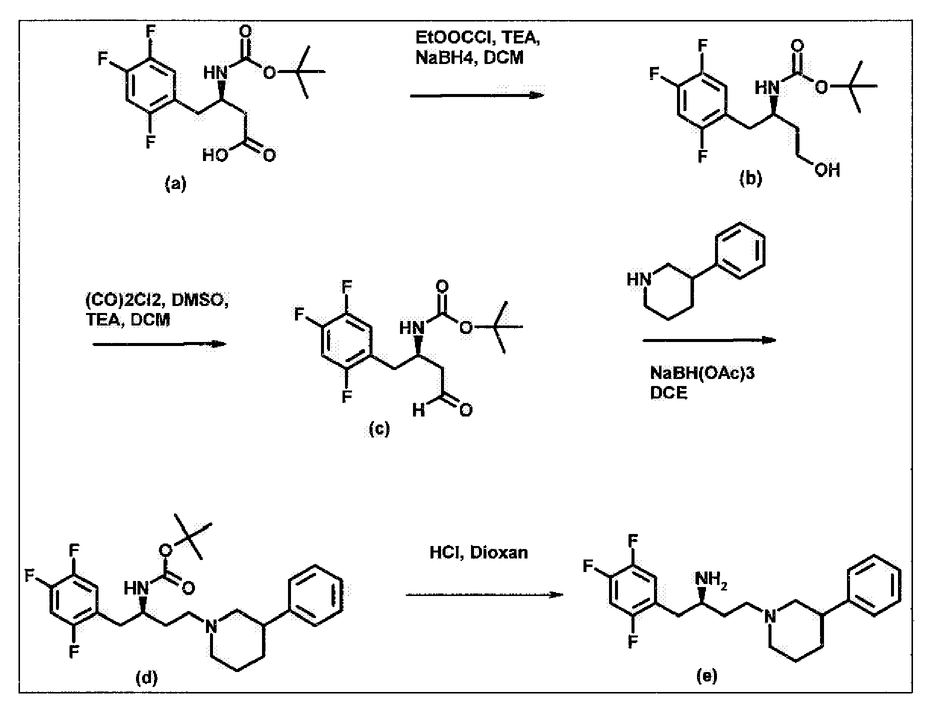

The reduction of the acid (a) to the corresponding alcohol (b) can be done using standard methods, e.g. sodium borohydride reduction of activated mixed anhydride, or using borane-THF complex.

Oxidation of the alcohol to the corresponding aldehyde (c) is typically performed by a Swern-type oxidation procedure using oxalyl chloride and DMSO.

The aldehyde then undergoes reductive aminations with cyclic secondary amines (piperidines or pyrrolidines). Standard conditions for reductive amination can be applied, e.g. sodium triacetoxyborohydride.

The resulting N-BOC-protected products (d) are deprotected using e.g. acidic conditions. Any salts can be formed, typically HCl, HBr, phosphate or salts of organic acids e.g. oxalate, maleate, fumarate, succinate, acetate or trifouoroacetate etc.

Racemates can be separated using standard methods (chromatography, diastereomeric salts, or via diastereotopic derivatization) as herein described.

### Example 1:

a) [(R)-3-Hydroxy-1-(2,4,5-trifluoro-benzyl)-propyl]-carbamic acid tert-butyl ester

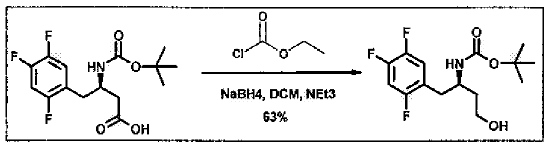

To a solution of (R)-3-tert-Butoxycarbonylamino-4-(2,4,5-trifluoro-phenyl)-butyric acid (2.5g, 7.5mmol) in dichloromethane (DCM) were added triethylamine (1.1ml) and ethyl chloroformate (0.78ml, 8.2mmol) at 0°C, the solution was stirred at rt during 15min., filtered and added to a solution of sodium borohydrate (424mg, 11.2mmol) in water (3.5ml) at 0°C. The raction mixture is stirred for 30min, then warmed to rt (2h stirring), then acidified with 1M HCl to pH 3 and extracted with ethyl acetate. After a short chromatography (Flashmaster) [(R)-3-Hydroxy-1-(2,4,5-trifluoro-benzyl)-propyl]-carbamic acid tert-butyl ester, (1.5g, 62% yield) was obtained a off-white powder.

Spectra:
MS (pos): 320 (M+1), 264 (M+1-tBu)
¹H-NMR (d₆-DMSO): 1.26 (s, 9H, tBu); 1.54 (m, 2H, CH₂-CH₂OH); 2.51 and 2.65-2.84 (m, 2H, CH₂-benzylic); 3.39 (m, 2H, CH₂-OH); 3.71 (m, 1H, CH-N); 4.39 (m, 1H, OH); 6.67 (d, 1H, NH); 7.28 (m, 1H, aromatic HC-ortho); 7.44 (m, 1H, aromatic HC-meta).

b) [(R)-3-Oxo-1-(2,4,5-trifluoro-benzyl)-propyl]-carbamic acid tert-butyl ester

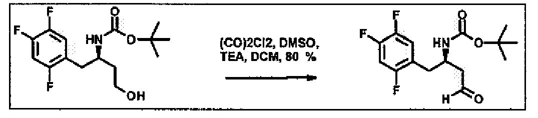

To a solution of oxalylchloride (0.674ml, 7.06mmol) in DCM (15ml) was added DMSO (0.67ml, 9.4mmol) dropwise at -78°C. After 15min the alcohol [(R)-3-Hydroxy-1-(2,4,5-trifluoro-benzyl)-propyl]-carbamic acid tert-butyl ester, (1.5g, 4.7mmol) was added. After 45min at -78°C triethylamine (3.27ml, 23.5mmol) was added, then stirred at rt for 4h. The reaction was quenched with water, extracted with DCM, washed with aqueous NaHSO4 and NaHCO3 solutions, The organic layers were dried and evaporated. Flash chromatography on silica gel (hexane-AcOEt, 6:4) resulted in [(R)-3-Oxo-1-(2,4,5-trifluoro-benzyl)-propyl]-carbamic acid tert-butyl ester (1.2g, yield: 80%) as yellowish solid.
Spectra:
MS (neg): 316 (M-1)
¹H-NMR (d₆-DMSO): 1.26 (s, 9H, tBu); 2.50-2.53 and 2.54-2.63 (m, 2H, CH₂-CHO); 2.54-2.63 and 2.70-2.94 (m, 2H, CH₂-benzylic); 4.16 (m, 1H, CH-N); 6.89 (d, 1H, NH); 7.31 (m, 1H, aromatic HC-ortho); 7.47 (m, 1 H, aromatic HC-meta); 9.56 (s, 1H, CHO).

c) [(R)-3-(3-phenyl-piperidin-1-yl)-1-(2,4,5-trifluoro-benzyl)-propyl]-carbamic acid tert-butyl ester

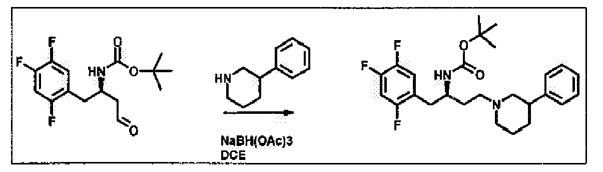

To [(R)-3-oxo-1-(2,4,5-trifluoro-benzyl)-propyl]-carbamic acid tert-butyl ester (0.6g, 1.89mmol) and commercial 3-phenyl-piperidine (458mg, 2.84mmol) in dichloroethylene (7.5ml) was added sodium triacetoxyborohydrate (1.06g, 4.75mmol) and stirred for 16h at rt.. Workup by extraction with ethyl acetate and water. The organic layers were dried and evaporated and the product purified by flash chromatography on silica gel (DCM). [(R)-3-(3-phenyl-piperidin-1-yl)-1-(2,4,5-trifluoro-benzyl)-propyl]-carbamic acid tert-butyl ester (0.77g, 1.66mmol, 88%) was obtained as white solid.

Spectra:
MS (pos): 463 (M+1)
¹H-NMR (d₆-DMSO): 1.25 (s, 9H, tBu); 1.33-1.46 (m, 1H, CH₂-piperidine); 1.46-1.56 (m, 1H, CH₂-piperidine); 1.58 (m, 2H, N(BOC)-CH₂CH₂-N(piperidine)); 1.64-1.72 (m, 1 H, CH₂-piperidine); 1.74-1.82 (m, 1 H, CH₂-piperidine); 1:83-1.97 (m, 2H, CH₂-piperidine); 2.29 (m, 2H, CH₂N(piperidine)); 2.50-2.55 (m, 1 H, CH₂-benzylic); 2.66 (m, 1H, CH-piperidine-phenyl); 2.72-2.78 (m, 1 H, CH₂-benzylic); 2.77-2.85 (m, 2H, CH₂-Piperidine); 3.64 (m, 1H, CH-NHBOC); 6.73 (d, 1H, NH-BOC); 7.07-7.37 (m, 6H, aromatic protons at phenyl and HC-ortho in fluoro-aromat); 7.44 (m, 1H, fluoro-aromat HC-meta)).

d) (R)-3-(3-Phenyl-piperidin-1-yl)-1-(2,4,5-trifluoro-benzyl)-propylamine

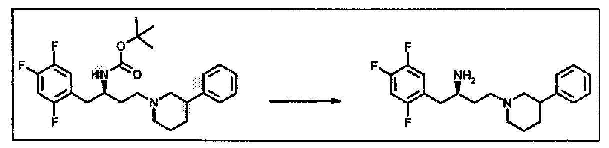

[(R)-3-(3-Phenyl-piperidin-1-yl)-1-(2,4,5-trifluoro-benzyl)-propyl]-carbamic acid tert-butyl ester (0.77g, 1.66mmol) was dissolved in dioxane (5ml), and for 5h treated with 4M aqueous hydrochloric acid (5ml). Evaporation of the solvents gives directly the dihydrochloride salt of (R)-3-(3-Phenyl-piperidin-1-yl)-1-(2,4,5-trifluoro-benzyl)-propylamine (730mg, 1.66mmol, 99%) and is lyophilized as white powder.

Spectra:
MS (pos): 363 (M+1)
¹H-NMR (d₆-DMSO): (as HCl-salt) 1.64-1.72 (m, 1H, CH2-piperidine); 1.85-1.91 (m, 1H, CH2-piperidine); 1.91-2.02 (m, 2H, CH2-piperidine); 2.02-2.11 (m, 2H, NH-CH₂CH₂-N(piperidine)); 2.88-2.94 (m, 2H, CH₂-benzylic); 2.93-2.97 (m, 1H, CH2-piperidine); 3.00-3.11 and 3.16-3.46 (m, 2H, CH2-piperidine); 3.16-3.23 (m, 1 H, CH-piperidine, benzylic); 3.23-3.31 (m, 2H, CH₂N(piperidine)); 3.47-3.50 (m, 1 H, CH2-piperidine); 3.59 (m, 1H, CH-NH2); 7.26-7.27 (m, 2H, phenyl (ortho)); 7.26-7.29 (m, 1 H, phenyl(para)); 7.36 (m, 2H, phenyl(meta)); 7.56 (m, 1H, fluoro-aromat HC-meta); 7.66 (m, 1 H, fluoro-aromat HC-ortho); 8.36 (s br, 1 H, NH3); 10.76 (s br, 1 H, NH-piperidine).

### Library formation

Using the reaction hereinbefore described, the following derivatives may be prepared by.
1. Reductive amination of N-Boc protected aldehyde (i)
2. Deprotection to yield final products, see example (ii)

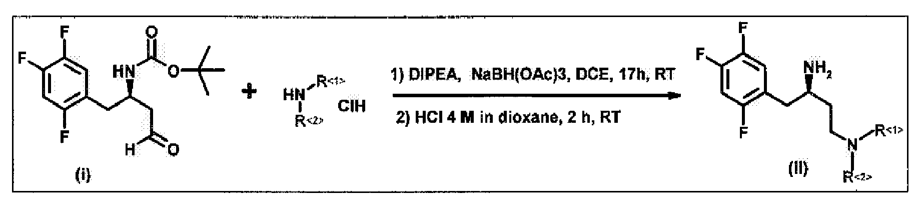

The reaction may be performed as parallel array.

Products may be isolated as salts, e.g. dihydrochloride salts.

The following table represents a library of compounds made by the aforementioned method(s):

| **Compound Number** | **Compound name** | **Empirical Formula** | **MW free base** | **MS [M+]** | **HPLC PURITY** | **HPLC retention** |
|---|---|---|---|---|---|---|
| **1** | (R)-3-(3-Phenyl-piperidin-1-yl)-1-(2,4,5-trifluorobenzyl)-propylamine | C₂₁H₂₅F₃N₂ | 362.44 | 363 | 99% | 1.30 min |
| **2** | (R)-3-[3-(4-Methoxyphenyl)-piperidin-1-yl]-1-(2,4,5-trifluorobenzyl)-propylamine | C₂₂H₂₇F₃N₂O | 392.47 | 393 | 99% | 1.22 min |
| **3** | (R)-3-[3-(3-Methoxyphenyl)-piperidin-1-yl]-1-(2,4,5-trifluorobenzyl)-propylamine | C₂₂H₂₇F₃N₂O | 392.47 | 393 | 99% | 1.19 min |
| **4** | (R)-3-[3-(2-Methoxyphenyl)-piperidin-1-yl]-1-(2,4,5-trifluorobenzyl)-propylamine | C₂₂H₂₇F₃N₂O | 392.47 | 393 | 99% | 1.25 min |
| **5** | (R)-1-(2,4,5-Trifluoro-benzyl)-3-[3-(4-trifluoromethylphenyl)-piperidin-1-yl]-propylamine | C₂₂H₂₄F₆N₂ | 430.44 | 431 | 99% | 1.41 min |
| **6** | (R)-1-(2,4,5-Trifluoro-benzyl)-3-[3-(3-trifluoromethylphenyl)-piperidin-1-yl]-propylamine | C₂₂H₂₄F₆N₂ | 430.44 | 431 | 99% | 1.30 min |
| **7** | (R)-1-(2,4,5-Trifluoro-benzyl)-3-[3-(2-trifluoromethylphenyl)-piperidin-1-yl]-propylamine | C₂₂H₂₄F₆N₂ | 430.44 | 431 | 99% | 1.28 min |
| **8** | (R)-3-(3-p-Tolylpiperidin-1-yl)-1-(2,4,5-trifluoro-benzyl)-propylamine | C₂₂H₂₇F₃N₂ | 376.47 | 377 | 99% | 1.26 min |
| **9** | (R)-3-(3-m-Tolylpiperidin-1-yl)-1-(2,4,5-trifluoro-benzyl)-propylamine | C₂₂H₂₇F₃N₂ | 376.47 | 377 | 99% | 1.24 min |
| **10** | (R)-3-(3-o-Tolylpiperidin-1-yl)-1-(2,4,5-trifluoro-benzyl)-propylamine | C₂₂H₂₇F₃N₂ | 376.47 | 377 | 99% | 1.14 min |
| **11** | (R)-3-[3-(4-Fluorophenyl)-piperidin-1-yl]-1-(2,4,5-trifluoro-benzyl)-propylamine | C₂₁H₂₄F₄N₂ | 380.43 | 381 | 99% | 1.15 min |
| **12** | (R)-3-[3-(3-Fluorophenyl)-piperidin-1-yl]-1-(2,4,5-trifluoro-benzyl)-propylamine | C₂₁H₂₄F₄N₂ | 380.43 | 381 | 99% | 1.21 min |
| **13** | (R)-3-[3-(2-Fluorophenyl)-piperidin-1-yl]-1-(2,4,5-trifluoro-benzyl)-propylamine | C₂₁H₂₄F₄N₂ | 380.43 | 381 | 99% | 1.56 min |
| **14** | (R)-3-(3-Phenyl-pyrrolidin-1-yl)-1-(2,4,5-trifluoro-benzyl)-propylamine | C₂₀H₂₃F₃N₂ | 348.41 | 349 | 99% | 1.16 min |
| **15** | (R)-3-[3-(3-Chlorophenyl)-pyrrolidin-1-yl]-1-(2,4,5-trifluoro-benzyl)-propylamine | C₂₀H₂₂ClF₃N₂ | 382.86 | 383 | 99% | 1.24 min |
| **16** | (R)-3-[3-(2-Chlorophenyl)-pyrrolidin-1-yl]-1-(2,4,5-trifluoro-benzyl)-propylamine | C₂₀H₂₂CIF₃N₂ | 382.86 | 383 | 99% | 1.21 min |
| **17** | (R)-3-[3-(4-Methoxyphenyl)-pyrrolidin-1-yl]-1-(2,4,5-trifluoro-benzyl)-propylamine | C₂₁H₂₅F₃N₂O | 378.44 | 379 | 99% | 1.16 min |
| **18** | (R)-3-[3-(3-Methoxyphenyl)-pyrrolidin-1-yl]-1-(2,4,5-trifluoro-benzyl)-propylamine | C₂₁H₂₅F₃N₂O | 378.44 | 379 | 99% | 1.18 min |
| **19** | (R)-3-[3-(2-Methoxy-phenyl)-pyrrolidin-1-yl]-1-(2,4,5-tifluoro-benzyl)-propylamine | C₂₁H₂₅F₃N₂O | 378.44 | 379 | 99% | 1.19 min |
| **20** | (R)-1-(2,4,5-Trifluoro-benzyl)-3-[3-(4-trifluoromethylphenyl)-pyrrolidin-1-yl]-propylamine | C₂₁H₂₂F₆N₂ | 416.41 | 417 | 99% | 1.29 min |
| **21** | (R)-3-[3-(4-Fluoro-phenyl)-pyrrolidin-1-yl]-1-(2,4,5-trifluoro-benzyl)-propylamine | C₂₀H₂₂F₄N₂ | 366.41 | 367 | 99% | 1.19 min |
| **22** | (R)-3-[3-(3-Fluorophenyl)-pyrrolidin-1-yl]-1-(2,4,5-trifluoro-benzyl)-propylamine | C₂₀H₂₂F₄N₂ | 366.41 | 367 | 99% | 1.19 min |
| **23** | (R)-3-[3-(2-Fluorophenyl)-pyrrolidin-1-yl]-1-(2,4,5-trifluoro-benzyl)-propylamine | C₂₀H₂₂F₄N₂ | 366.41 | 367 | 99% | 1.17 min |
| **24** | (R)-3-Piperidin-1-yl-1-(2,4,5-trifluoro-benzyl)-propylamine | C₁₅H₂₁F₃N₂ | 286.34 | 287 | >90% by NMR | 0.62 min |
| **25** | (R)-3-(3-Methyl-piperidin-1-yl)-1-(2,4,5-trifluoro-benzyl)-propylamine | C₁₆H₂₃F₃N₂ | 300.37 | 301 | 99% | 0.87 min |
| **26** | (R)-3-(3-Methoxymethyl-piperidin-1-yl)-1-(2,4,5-trifluoro-benzyl)-propylamine | C₁₇H₂₅F₃N₂O | 330.4 | 331 | 99% | 0.76 min |
| **27** | (R)-3-(3-Benzo[1,3]dioxol-5-yl-piperidin-1-yl)-1-(2,4,5-trifluoro-benzyl)-propylamine | C₂₂H₂₅F₃N₂O₂ | 406.45 | 407 | 99% | 1.21 min |
| **28** | (R)-3-Pyrrolidin-1-yl-1-(2,4,5-trifluoro-benzyl)-propylamine | C₁₄H₁₉F₃N₂ | 272.32 | 273 | >90% by NMR | 0.51 min |
| **29** | R)-3-[(R)-3-(4-Fluoro-phenyl)-pyrrolidin-1-yl]-1-(2,4,5-trifluoro-benzyl)-propylamine | C₂₀H₂₂F₄N₂ | 366.41 | 367 | 99% | 1.20 min |

### Specification of HPLC-MS system:

Agilent 1100 LC chromatographic system with Micromass ZMD MS detection. A binary gradient composed of A (water containing 5 % acetonitrile and 0.05% trifluoroacetic acid) and B (acetonitrile containing 0.045% trifluoroacetic acid) is used as a mobile phase on a Waters X TerraTM C-18 column (30 x 3 mm, 2.5mm particle size) as a stationary phase.

The following elution profile is applied: a linear gradient of 1.5 minutes at a flow rate of 0.6 ml/min from 10% of B to 95% of B, followed by an isocratic elution of 0.5 minutes at a flow rate of 0.7 ml/min of 95% of B, followed by an isocratic elution of 0.5 minutes at a flow rate of 0.8 ml/min of 95% of B, followed by a linear gradient of 0.2 minutes at a flow rate of 0.8 ml/min from 95% of B to 10% of B, followed by an isocratic elution of 0.2 minutes at a flow rate of 0.7 ml/min of 10% of B.

### Biological Testing

For compound screening, the assay employed measures the activity of recombinant human DPP-4 to cleave the synthetic fluorogenic substrate (H-Ala-Pro)2-Rh110. The reaction was run in 384- well plates. Compound solutions in assay buffer were pre-incubated with the human recombinant DPP-4 and the reaction was initiated by the addition of the substrate. The product of the reaction was quantified by measuring the fluorescence intensity using Tecan Ultraplate reader. For each compound concentration tested, the percent of inhibition was calculated.

For the determination of IC₅₀ and/or % of inhibition values, the assay was performed at room temperature in 384-well plates using a TECAN Ultra plate reader. Total assay volume was 30 µl. Test compounds were dissolved in 90 % (v/v) DMSO/water and diluted in water containing 0.05 % (w/v) CHAPS to 3-times the desired assay concentration. For the assay, 10 µl water/CHAPS (± test compound) were added per well, followed by the addition of 10 µl hDPP4 solution (diluted with 1.5x assay buffer, *i.e.* 37.5 mM Tris/HCl, pH 7.4, 210 mM NaCl, 15 mM KCl, and 0.05 % (w/v) CHAPS). The final assay concentrations were 10 pM for hDPP4 according to the enzyme concentrations determined by the Bradford method. After 1 hour of pre-incubation at room temperature, the reaction was started by the addition of 10 µl substrate solution (substrate dilutes in 1.5x assay buffer, final substrate concentration was 10 µM). The effect of the compound on the enzymatic activity was obtained from the linear progression curves and determined from two readings, the first one taken directly after the addition of substrate (t = 0 min) and the second one after 1 hour (t = 60 min). The apparent inhibition constant, IC₅₀, was calculated from the plot of percentage of inhibition vs. inhibitor concentration using non-linear regression analysis software (XLfit, Vers. 4.0; ID Business Solution Ltd., Guildford, Surrey, UK).

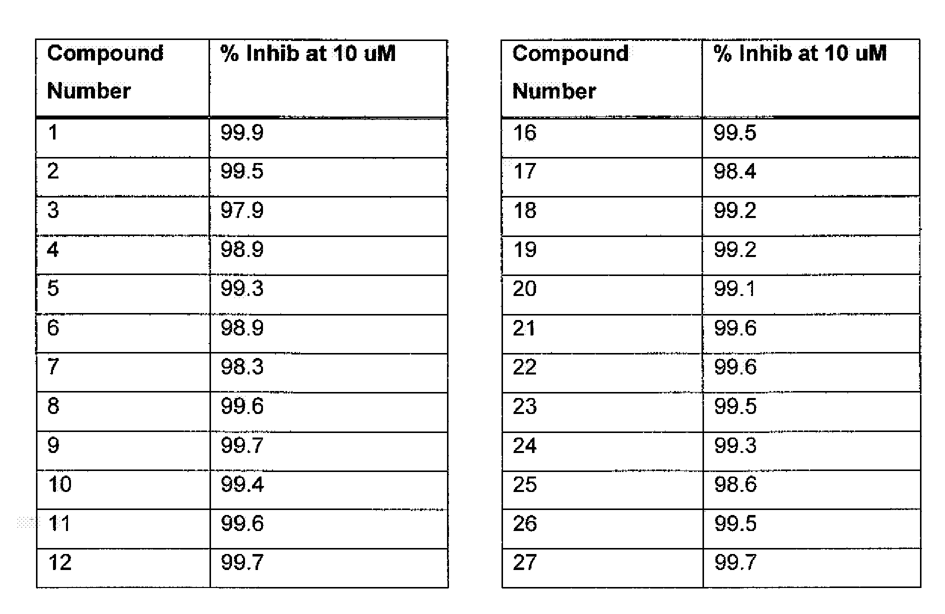

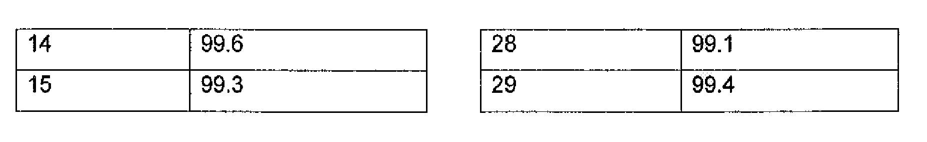

### Uses

The present invention is directed to the use of the compounds disclosed herein as inhibitors of serine proteases, including dipeptidyl peptidases, such as dipeptidyl peptidase-IV and dipeptidyl peptidase-II, for the prevention, delay of progression or the treatment of the human diseases in which serine peptidases are involved.

In particular, the present invention is directed to the use of the compounds disclosed herein as inhibitors of dipeptidyl peptidase-IV enzyme activity. These compounds may be useful in human diseases including but are not limited to autoimmune diseases such as multiple sclerosis, neurological disorders, dementia, neurodegenerative diseases, mood disorders, other psychiatric conditions, diabetes in particular type II diabetes and related conditions, arthritis, obesity, osteoporosis and conditions of impaired glucose tolerance.

DPP-IV enzyme exists as both soluble form circulating in body fluids (blood and cerebrospinal fluid) and as a cell surface protein (called T cell activation marker or CD26) and has been implicated in a wide range of biological functions. DPP-IV can cleave a number of immunoregulatory, endocrine, and neuropeptides. This has suggested a potential role for this peptidase in a variety of disease processes in humans or other species.

Effects of DPP-IV inhibitors may include both direct inhibition of DPP-IV enzymatic activity which has as a consequence to prevent the cleavage of its substrates such neuropeptides and indirect effects through the blockade of activation of T lymphocytes which express CD26/DPP-IV.

Accordingly, the compounds of the present invention are useful for the prevention or treatment of diseases, disorders and conditions associated with serine proteases, such as DPP, for example.

The compounds of the present invention are useful for treating disorders such as:
o Nervous system (NS) and non-NS autoimmune diseases, immunological disorders and/or inflammatory diseases, such as multiple sclerosis, inflammatory bowel disease and rheumatoid arthritis, Graves' disease, Hashimoto's thyroiditis, transplantation.
o Neurodegenerative and neurologic disorders, such as dementia, cognitive disorders such as cognition and/or learning impairment, impaired short- or long-term memory conditions, and impaired learning conditions, other nervous system diseases characterized by brain insulin resistance, brain abnormal glucose utilization and/or metabolism and/or by changes in the level of polypeptides substrates of DPP-IV and/or DPP-II enzymes. Further examples are chronic neurodegenerative diseases, including Alzheimer's disease, Parkinson's disease, Huntington's disease, amyotrophic lateral sclerosis, and acute neurodegenerative diseases, such as stroke and spinal cord injuries.
o Psychiatric diseases, such as anxiety, depression, schizophrenia, other mood disorders, and other nervous system diseases where DPP-II and/or DPP-4/CD26 are implicated and/or where changes in the levels of substrates of these enzymes, including neuropeptides, hormones may occur.
o Diabetes including Type II diabetes (where a number of DPP-IV inhibitors showed beneficial effects both in animal models of diabetes and in clinical studies in patients with diabetes) and metabolic and other conditions linked directly or indirectly to diabetes including hyperglycemia, low glucose tolerance, insulin resistance, obesity, lipid disorders, dyslipidemia, hyperlipidemia, hypertriglyceridemia, hypercholesterolemia, low HDL levels, high LDL levels, atherosclerosis and its sequelae, vascular restenosis, irritable bowel syndrome, inflammatory bowel disease, including Crohn's disease and ulcerative colitis, other inflammatory conditions, pancreatitis, obesity, neuropathies, neurologic disorders, neurodegenerative diseases, psychiatric diseases, retinopathy, nephropathy, Syndrome X, ovarian hyperandrogenism (polycystic ovarian syndrome), and other disorders where insulin resistance is a component and/or endogenous substrates of DPP-IV and/or DPP-II enzymes are degraded.
o Other indications
   o Benign Prostatic Hypertrophy
   o Sperm motility/male contraception
   o Gingivitis
   o Osteoporosis: GIP receptors are present in osteoblasts.
   o Pain
   o Asthma
   o Obeisety
   o Growth Hormaone Deficiency
   o Intestinal Injury
   o HIV infection or AIDS
   o Hematopoiesis
   o Tumor Invasion and Metastasis
   o Skin disorders
   o Neurogenic inflammation
   o Heart failure
   o Atherosclerosis
   o Hypertension

The subject compounds are useful in a method of inhibiting the dipeptidyl peptidase-IV enzyme in a patient such as a mammal in need of such inhibition comprising the administration of an effective amount of the compound.

The present invention is further directed to a method for the manufacture of a medicament for inhibiting dipeptidyl peptidase-IV enzyme activity in humans and animals comprising combining a compound of the present invention with a pharmaceutical carrier or diluent.

The present invention also relates to compositions comprising a compound of formula (I).

The term "composition" as used herein is intended to encompass a product comprising the specified ingredients in the specified amounts, as well as any product which results, directly or indirectly, from combination of the specified ingredients in the specified amounts. Such term in relation to pharmaceutical composition, is intended to encompass a product comprising the active ingredient (s), and the inert ingredient (s) that make up the carrier, as well as any product which results, directly or indirectly, from combination, complexation or aggregation of any two or more of the ingredients, or from dissociation of one or more of the ingredients, or from other types of reactions or interactions of one or more of the ingredients.

Accordingly, the pharmaceutical compositions of the present invention encompass any composition made by admixing a compound of the present invention and a pharmaceutically acceptable carrier. By "pharmaceutically acceptable" it is meant the carrier, diluent or excipient must be compatible with the other ingredients of the formulation and not deleterious to the recipient thereof.

The active agents of the invention may be administered by any conventional route, in particular enterally, preferably orally, e.g. in the form of tablets or capsules, or parenterally, e.g. in the form of injectable solutions or suspensions.

The compounds of the invention can also be combined with other drugs "combined preparations". The compounds of the present invention may be administered alone or in combination with the systemic or localco-administration of one or more additional agents. Such agents include atypical antipsychotic drugs such as clozapine, olanzapine, risperidone, typical antipsychotic drugs such as haloperidol, Anti-epileptic Drugs, nootropics, immunosuppressants, growth factors, preservatives, ventricle wall permeability increasing factors, stem cell mitogens, survival factors, glial lineage preventing agents, anti-apoptotic agents, anti-stress medications, neuroprotectants, and anti-pyrogenics and other drugs suitable for: the treatment of Neurological Disorders, affective and attention disorders, the treatment of neurological / psychiatric disorders, the treatment of ocular disorders, in particular myopia, suitable for the treatment of pain, especially neuropathic pain, the treatment of dementia, treatment of acute brain lesions, anti-diabetic agents, hypolipidemic agents, anti-obesity or appetite-regulating agents, anti-hypertensive agents, HDL-increasing agents, cholesterol absorption modulators, Apo-A1 analogues and mimetics, thrombin inhibitors, aldosterone inhibitors, inhibitors of platelet aggregation, estrogen, testosterone, selective estrogen receptor modulators, selective androgen receptor modulators, chemotherapeutic agents, and 5-HT₃ or 5-HT₄ receptor modulators.

The term "a combined preparation", as used herein defines especially a "kit of parts" in the sense that the first and second active ingredient as defined above can be dosed independently or by use of different fixed combinations with distinguished amounts of the ingredients, i.e., simultaneously or at different time points. The parts of the kit of parts can then, e.g., be administered simultaneously or chronologically staggered, that is at different time points and with equal or different time intervals for any part of the kit of parts. Very preferably, the time intervals are chosen such that the effect on the treated disease in the combined use of the parts is larger than the effect which would be obtained by use of only any one of the active ingredients. The ratio of the total amounts of the active ingredient 1 to the active ingredient 2 to be administered in the combined preparation can be varied, e.g., in order to cope with the needs of a patient sub-population to be treated or the needs of the single patient which different needs can be due to age, sex, body weight, etc. of the patients. Preferably, there is at least one beneficial effect, e.g., a mutual enhancing of the effect of the first and second active ingredient, in particular a synergism, e.g. a more than additive effect, additional advantageous effects, less side effects, a combined therapeutical effect in a non-effective dosage of one or both of the first and second active ingredient, and especially a strong synergism the first and second active ingredient.

It will be understood that in the discussion of methods, references to the active ingredients are meant to also include the pharmaceutically acceptable salts. If these active ingredients have, for example, at least one basic center, they can form acid addition salts. Corresponding acid addition salts can also be formed having, if desired, an additionally present basic center. The active ingredients having an acid group (for example COOH) can also form salts with bases. The active ingredient or a pharmaceutically acceptable salt thereof may also be used in form of a hydrate or include other solvents used for crystallization.

In particular, a therapeutically effective amount of each of the active ingredients of a combination may be administered simultaneously or sequentially and in any order, and the components may be administered separately or as a fixed combination. For example, the method of treatment of diseases according to the invention may comprise (i) administration of the first active ingredient in free or pharmaceutically acceptable salt form and (ii) administration of the second active ingredient in free or pharmaceutically acceptable salt form, simultaneously or sequentially in any order, in jointly therapeutically effective amounts, preferably in synergistically effective amounts, e.g. in daily dosages corresponding to the amounts described herein. The individual active ingredients of the combination can be administered separately at different times during the course of therapy or concurrently in divided or single combination forms. Furthermore, the term administering also encompasses the use of a prodrug of an active ingredient that convert *in vivo* to the active ingredient. The instant invention is therefore to be understood as embracing all such regimes of simultaneous or alternating treatment and the term "administering" is to be interpreted accordingly.

The pharmaceutical compositions according to the invention can be prepared in a manner known per se and are those suitable for enteral, such as oral or rectal, and parenteral administration to mammals (warm-blooded animals), including man, comprising a therapeutically effective amount of at least one pharmacologically active ingredient, alone or in combination with one or more pharmaceutically acceptable carries, especially suitable for enteral or parenteral application. The preferred route of administration of the dosage forms of the present invention is orally.

The novel pharmaceutical composition contain, for example, from about 10% to about 100%, preferably from about 20% to about 60%, of the active ingredients. Pharmaceutical preparations for the combination therapy for enteral or parenteral administration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. It will be appreciated that the unit content of active ingredient or ingredients contained in an individual dose of each dosage form need not in itself constitute an effective amount since the necessary effective amount can be reached by administration of a plurality of dosage units.

In, preparing the compositions for oral dosage form, any of the usual pharmaceutical media may be employed, such as, for example, water, glycols, oils or alcohols; or carriers such as starches, sugars, microcristalline cellulose, diluents, granulating agents, lubricants, binders, disintegrating agents and the like in the case of oral solid preparations such as, for example, powders, capsules and tablets. Because of their ease of administration, tablets and capsules represent the most advantageous oral dosage unit form in which case solid pharmaceutical carriers are obviously employed.

## Claims

1. A compound of the general formula (I): 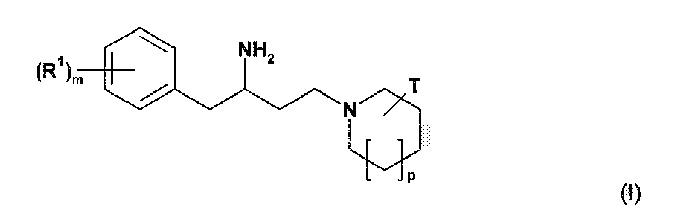 wherein
m is 0,1,2,3,4 or 5;
p is 0 or 1;
each R¹ is independently selected from halogen, alkyl, haloalkyl, alkoxy, haloalkyloxy; cycloalkyl; and
T is selected from optionally substituted aryl, optionally substituted alkyl, hydrogen, halogen, alkoxy, haloalkyloxy, COO-alkyl, aryloxy;
and pharmaceutically acceptable salts and N-oxides thereof.

2. The compound of claim 1, wherein T is substituted aryl.

3. The compound of any one of claims 1 or 2 wherein p is 1.

4. The compound of any preceding claim having the formula (II): 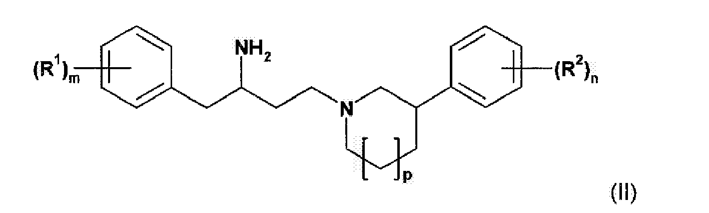 wherein:
n and m are each independently 0,1,2,3,4 or 5;
p is 0 or 1;
each R¹ is independently selected from halogen, alkyl, haloalkyl, alkoxy, haloalkyloxy; cycloalkyl; and
each R² is independently selected from halogen, alkyl, haloalkyl, alkoxy, haloalkyloxy, COO-alkyl, aryloxy; and
where n is 2 or more, two R² substituents may together form a 5 or 6 membered ring;
and pharmaceutically acceptable salts and N-oxides thereof.

5. A compound of claim 1, having the formula (III) 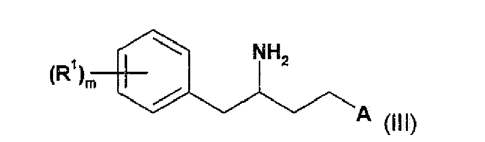 where A is selected from

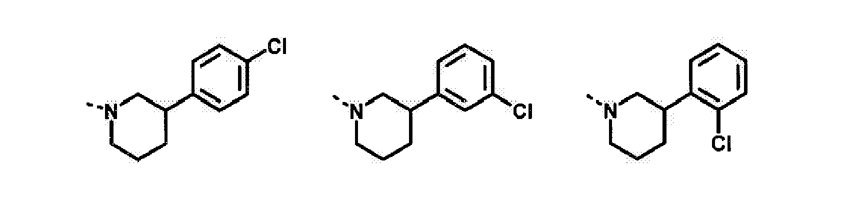

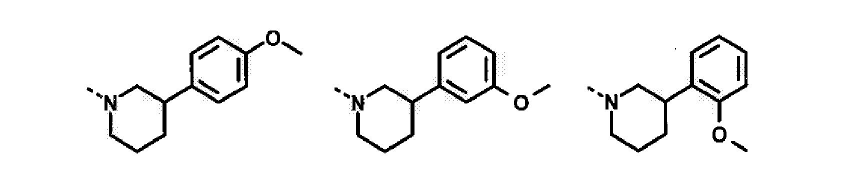

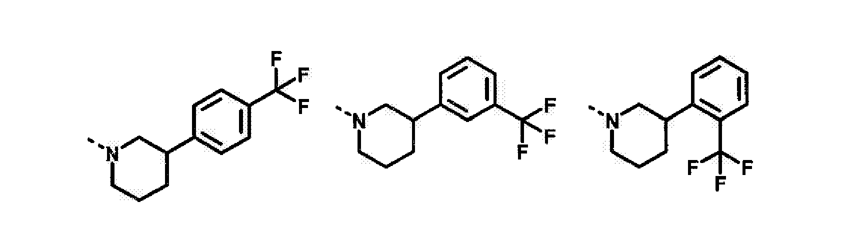

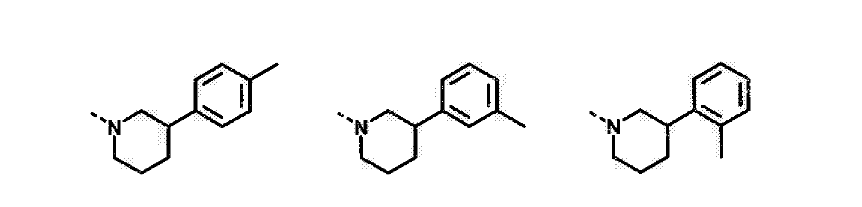

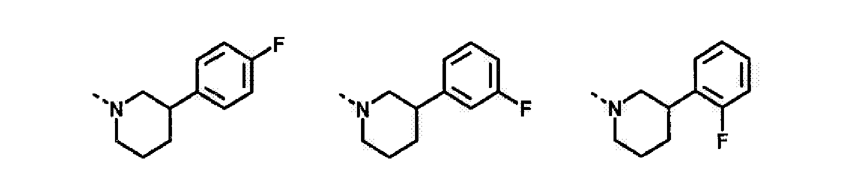

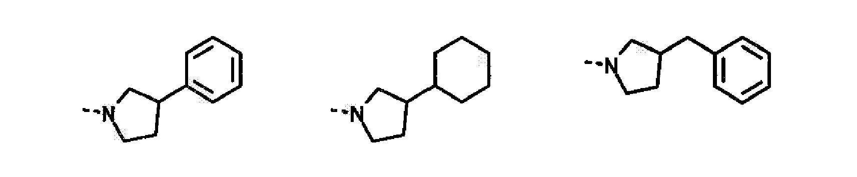

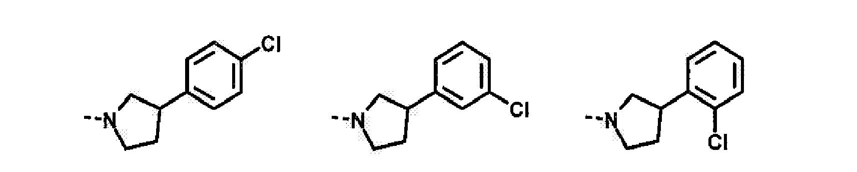

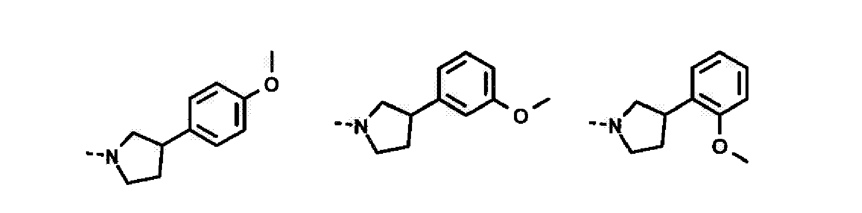

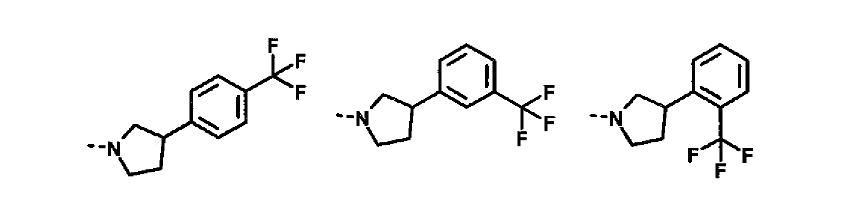

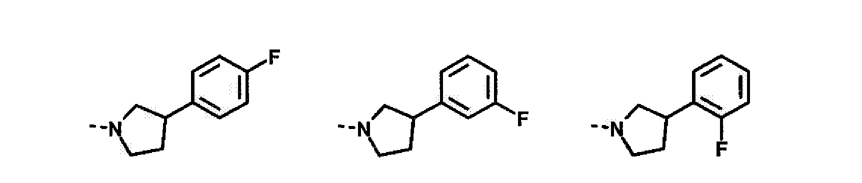

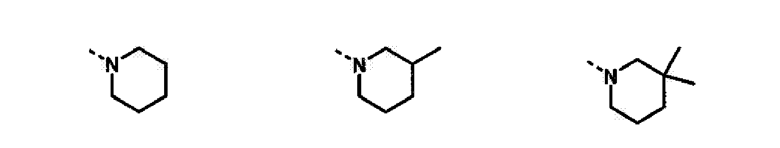

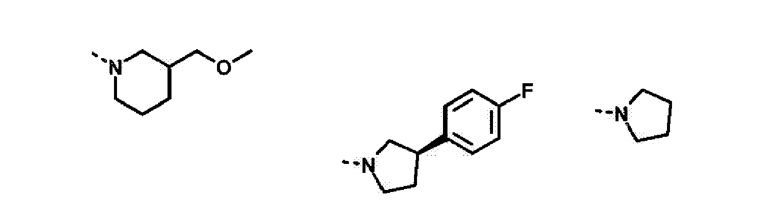

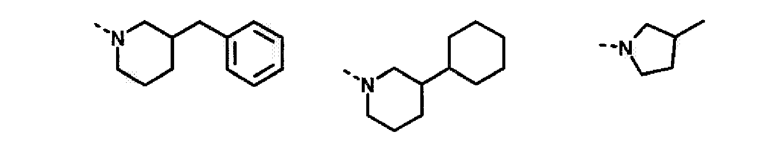

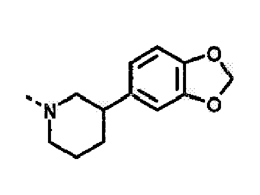

6. The compound of any preceding claim, wherein R¹ is halogen.

7. The compound of any preceding claim, wherein m is 3.

8. The use of a compound of any preceding claim as a pharmaceutical.

9. A pharmaceutical composition comprising a compound of any one of claims 1 to 7 and one or more pharmaceutically acceptable diluents or carriers.

10. A pharmaceutical combination comprising a compound of any one of claims 1 to 7 and one or more co-agents, e.g. a second drug agent.

11. The use of a compound of any preceding claim in the manufacture of a medicament for treating a serine protease disorder.

12. The use of claim 11, wherein the disorder is a DPP-related disorder.

13. The use of a compound of any preceding claim in the manufacture of a medicament for treating autoimmune diseases, immunological disorders, inflammatory diseases, Neurodegenerative and neurologic disorders, Psychiatric diseases, Diabetes, Pain or a skin disorder.

14. A method for modulating a serine protease- related disease in a subject comprising administering a composition comprising a compound of any one of claims 1 to 7 in an a therapeutically effective amount.
